# EUROPEAN PATENT APPLICATION

(11) **EP 4 276 196 A1**
(43) Date of publication of application: **15.11.2023**
(21) Application number: 22172519.5
(22) Date of filing: 10.05.2022
(51) Int. Cl.: C12Q 1/6848

(54) **DECOY-OLIGONUCLEOTIDES IN NUCLEIC ACID DETECTION METHODS**

(71) Applicant: PHILIPPS-UNIVERSITÄT MARBURG, 35037 Marburg (DE)
(72) Inventor: Adhikary, Till, 35287 Amöneburg (DE); Chung, Ho-Ryun, 35043 Marburg (DE)
(74) Representative: Durm Patentanwälte PartG mbB

(57) **Abstract**

The invention relates to reagent kits comprising a decoy-oligonucleotide and a probe, wherein the probe comprises a cleavage site, and a binding site hybridisable to a pre-determined nucleotide sequence and the decoy-oligonucleotide comprises a 3'-OH group, at least one mismatch base compared to the binding site of the probe. The invention further relates to methods for detecting the presence of a pre-determined nucleotide sequence in a sample, the method comprising the steps of adding said reagent kit additionally comprising at least one DNA primer; adding one or more enzyme(s) providing activities of DNA polymerase activity and strand-displacement activity to the sample to be analysed for the presence of the pre-determined nucleotide sequence; amplifying the pre-determined nucleotide sequence; and detecting whether amplification products are present in the sample, wherein presence of the amplification product in the sample is indicative of the presence of the pre-determined nucleotide sequence in the sample.

## Description

The invention relates to reagent kits comprising a decoy-oligonucleotide and a probe, wherein the probe comprises a cleavage site, and a binding site hybridisable to a pre-determined nucleotide sequence and the decoy-oligonucleotide comprises a 3'-OH group, at least one mismatch base compared to the binding site of the probe. The invention further relates to methods for detecting the presence of a pre-determined nucleotide sequence in a sample, the method comprising the steps of adding said reagent kit additionally comprising at least one DNA primer; adding one or more enzyme(s) providing activities of DNA polymerase activity and strand-displacement activity to the sample to be analysed for the presence of the pre-determined nucleotide sequence; amplifying the pre-determined nucleotide sequence; and detecting whether amplification products are present in the sample, wherein presence of the amplification product in the sample is indicative of the presence of the pre-determined nucleotide sequence in the sample.

DNA amplification methods are vulnerable to contamination and false positive results. This particularly affects diagnostics, which require a binary decision. A common source of false positive results is "carryover" contamination, where a nucleotide product (amplicon) from a prior assay contaminates subsequent assays. The contaminant may be transmitted by a technician, an instrument or even via aerosol. In a "negative" sample, where the target nucleic acid is absent, the contaminant creates a false positive result. In a "positive" sample, where the target nucleic acid is present, the contaminant is co-amplified with the true target. Such co-amplification may distort a result of a quantitative assay, where exact amounts of the true target must be determined.

Practically, in diagnostics, carryover contamination is avoided by prevention of aerosol formation, frequent changing of gloves, sequential use of different workplaces for reaction setup and amplification, and further precautions. Maintaining sealed post-amplification tubes is another way to avoid carryover, however this requires more sophisticated detection methods based on colorimetric, luminometric or fluorimetric detection, necessitating appropriate instrumentation and complicating multiplexing.

Furthermore, reaction tubes must be opened in some detection methods. Overall, the avoidance of carryover contamination requires a disciplined and concentrated workflow, placing a high demand on resources and workplaces. Amplification methods that are particularly susceptible to carryover contamination are those conducted outside of the laboratory in resource-limited settings and for point-of-care analysis. These include isothermal amplification methods which do not require complex equipment for thermocycling. Recombinase polymerase amplification (RPA) is an isothermal DNA amplification method which, in contrast to the polymerase chain reaction (PCR) method, does not require cycling between different temperatures as described in US7666598B2. The primer oligonucleotides used in RPA methods limit the temperature to around 37-42 °C, which ensures binding only to complementary and near-complementary strands.

For the specific detection of a target sequence using RPA different tools have been developed and many are commercially available, including those described in US8017339B2. These use a third oligonucleotide ("probe") which binds to the amplicon sequence within the stretch between the primers. The probe is converted to a detection-competent state by an endonuclease (e.g. endonuclease IV from *E.coli,* which cleaves double-stranded DNA at abasic sites) present during amplification. Probe cleavage results in, for example, exposing a free 3' hydroxyl group which enables elongation or removal of a fluorescent dye proximal quencher, enabling fluorescence and thus detection. Detection of a specific amplicon therefore requires probe binding and cleavage. Multiplexing of samples is possible by using different dyes or combinations of labels on the probe.

Since RPA, like PCR, is an ultra-sensitive amplification protocol, it is particularly susceptible to carryover artefacts. Currently, techniques developed to mitigate contamination are limited. One method, described in US7666598B2 and US8017339B2, utilizes deoxyuridine triphosphate (dUTP), replacing deoxythymidine triphosphate (dTTP) in part or wholly for oligonucleotide synthesis. By using dUTP in combination with uracil deglycosylase (UDG) obtained from *E. Coli,* pre-determined breaking points are created in the sequences containing dUTP, which are subsequently degraded by the uracil-N-glycosylase (UNG) enzyme (A subclass of the UDG family of enzymes). UNG selectively degrades uracil-containing sequences leaving thymine-containing DNA intact. For this procedure to be effective, the UNG enzyme must be added prior to the start of a new measurement to destroy carryover DNA. Furthermore, it is necessary to remove UNG before the start of a new measurement, which is typically achieved by heat inactivation in PCR protocols. Heat inactivation is however precluded in isothermal methods, including RPA, due to the requirement of heat sensitive reagents and the need to minimize thermal cycling. The authors in US8062850B2 circumvented this issue by the addition of an UNG peptide inhibitor derived from *Bacillus* bacteriophages, which prevented UNG interference in subsequent amplifications. The authors noted however, that this protocol was sub-optimal and led to false-positive and false-negative results. Furthermore, this protocol is hampered by several additional problems including: 1) the generation of deoxyuridine monophophate (dUMP) upon hydrolysis of dUTP, which is incompatible with some polymerases used in amplification, and 2) the requirement for optimization of the enzymatic procedure for each individual assay.

A further method to eliminate carryover in DNA amplification techniques is UV irradiation of post-amplified samples. UV light induces dimerization between adjacent pyrimidine bases and results in strand breaks, thus preventing further processing of amplicons by polymerases. This method is disadvantaged by several factors including: 1) the need for specialized UV light equipment and trained staff, making it unamenable to point-of-care rapid tests and 2) the denaturing of UV sensitive substrates including some polymerases, which therefore need to be replenished after each amplification.

There is, therefore, a need in the art for improved reagents and methods able to overcome carryover effects in nucleic acid amplification techniques.

The solution to this technical problem is provided by the embodiments as defined herein below and as characterized in the claims.

Accordingly, the invention relates to, *inter alia,* the following embodiments:
1. A reagent kit comprising a decoy-oligonucleotide and a probe, wherein
   a) the probe comprises a cleavage site and a binding site hybridisable to a pre-determined nucleotide sequence; and
   b) the decoy-oligonucleotide comprises a 3'-OH group and at least one mismatch base compared to the binding site of the probe.
2. The reagent kit of embodiment 1, wherein the probe further comprises a label.
3. The reagent kit of embodiment 1 or 2, wherein the reagent kit comprises a larger number of decoy-oligonucleotides than probes.
4. The reagent kit of any one of embodiments 1 to 3, wherein the reagent kit comprises one or more DNA primer(s).
5. The reagent kit of embodiment 4, wherein the reagent kit comprises two or more DNA primers, wherein the binding site of the probe is between the binding site of at least two DNA primers on the pre-determined nucleotide sequence.
6. A method for detecting the presence of a pre-determined nucleotide sequence in a sample, the method comprising the steps of:
   (a) adding the reagent kit of any one of embodiments 1 to 5; wherein the reagent kit comprises at least one DNA primer;
   (b) adding one or more enzyme(s) providing activities of DNA polymerase activity and strand-displacement activity to the sample to be analysed for the presence of the pre-determined nucleotide sequence;
   (c) amplifying the pre-determined nucleotide sequence; and
   (d) detecting whether an amplification product is present in the sample,
   wherein presence of the amplification product in the sample is indicative of the presence of the pre-determined nucleotide sequence in the sample.
7. The method of embodiment 6, wherein amplifying the pre-determined nucleotide sequence comprises incubating the sample resulting from steps (a) and (b) at a fixed temperature.
8. The method of embodiment 7, wherein the fixed temperature is between 20 and 50°C, preferably between 35 and 45°C.
9. The method of any one of embodiments 6 to 8, wherein at least one enzyme providing activities of single-strand DNA-binding is added.
10. The method of any one of embodiments 6 to 9, wherein at least two DNA primers are added.
11. The method of any one of embodiments 6 to 10, wherein at least one of the DNA primers comprises a label.
12. The method of embodiment 11, wherein the DNA primer comprising a label which binds to the opposite strand than the probe.
13. The method of any one of embodiments 6 to 12, wherein the pre-determined nucleotide sequence comprises a pre-determined DNA sequence.
14. The method of embodiment 13, wherein the pre-determined DNA sequence comprises a sequence comprised in an extrachromosomal DNA sequence, preferably an extrachromosomal DNA sequence encoding a drug susceptibility-related property.
15. The method of any one of embodiments 6 to 12, wherein at least one of the added enzymes provides reverse transcriptase activities.
16. The method of embodiment 15, wherein the pre-determined nucleotide sequence is comprised in a coronavirus.

Accordingly, in a first embodiment, the invention relates to a reagent kit comprising a decoy-oligonucleotide and a probe, wherein a) the probe comprises a cleavage site and a binding site hybridisable to a pre-determined nucleotide sequence; and b) the decoy-oligonucleotide comprises a 3'-OH group and at least one mismatch base compared to the binding site of the probe.

The term "oligonucleotide", as used herein, refers to a nucleic acid that includes at least two nucleic acid monomer units (e.g., nucleotides such as DNA, RNA, PNA, LNA chimera, linked polymer as well as combinations thereof (e.g., an LNA/DNA chimera)), typically more than three monomer units, and more typically greater than ten monomer units. The exact size of an oligonucleotide generally depends on various factors, including the function or use of the oligonucleotide. Oligonucleotides can be prepared by any suitable method, including, but not limited to, isolation of an existing or natural sequence, DNA replication or amplification, reverse transcription, cloning and restriction digestion of appropriate sequences, or direct chemical synthesis by methods known in the art.

The term "probe", as used herein, refers to an oligonucleotide that is designed to sequence specifically hybridize to a pre-determined nucleic acid sequence of interest. The probe sequence described herein is an oligonucleotide comprising a cleavage site which enables detection upon hybridization. The oligonucleotide probe is typically labelled with a detectable moiety such as radioactive, fluorescent or chemiluminescent compound.

The term "decoy-oligonucleotide" or "decoy", as used herein, refers to an oligonucleotide which partially overlaps the probe binding site and competes with probe binding to the binding site of the amplicon. In particular, the partial overlap of the decoy-oligonucleotide occurs at either the 3'and/or 5'end of the cleavage site on the probe. The decoy-oligonucleotide comprises at least one mismatch base and a 3'-OH group compared to the probe. In some embodiments, the decoy-oligonucleotide comprises at least two mismatch bases compared to the probe. In some embodiments, the decoy-oligonucleotide comprises at least two mismatch bases, wherein at least one mismatch base is located at a position corresponding to one side of the cleavage site of the probe and at least one mismatch base is located at a position corresponding to the other side of the cleavage site of the probe. Both the probe and decoy-oligonucleotide comprise (a) overlapping sequence part(s). The required overlap of the probe and decoy sequence depends on various factors such as length, binding specificity, desired extent of competition and can be any overlap as long as the decoy competes with the binding of the probe. In some embodiments, the decoy has at least 50%, at least 60%, at least 70%, at least 80%, or at least 90% sequence identity compared to the sequence of the probe. In some embodiments, the decoy has at least 1, at least 2, at least 3, at least 4, or at least 5, mismatch base(s) compared to the sequence of the probe.

The term "mismatch base", as used herein, is a base-base mismatch, in that, at least one base of the sequence is non-complementary compared to the predetermined nucleotide sequence and probe. Preferably, the mismatch on the decoy is located at a site equivalent to the region of the cleavage site on the probe sequence. In a second preferred embodiment, the mismatch base is located on an equivalent location on either side (5'-end or 3'-end) of the cleavage site. Preferably, the bases at the 3'-end of the decoy-oligonucleotide are complementary to the pre-determined nucleotide sequence.

The term "cleavage site", as used herein, refers to a region of the probe sequence which is cleavable upon hybridisation to the target sequence. In particular, a region of the probe sequence that comprises, for example, an abasic residue, in particular a THF residue. The probe of the present invention is typically less than or equal to 150 nucleotides in length, typically less than or equal to 100 nucleotides, for example less than or equal to 80, 70, 60 or 50 nucleotides in length. The term, "abasic residue" or "abasic site" within an oligonucleotide, as used herein, refers to a molecular fragment within an oligonucleotide chain where the molecular fragment approximates the length of a ribofuranose or a deoxyribofuranose sugar in such a way that bases adjacent to the molecular fragment are separated from one another by the same, or effectively the same, distance as if a ribofuranose or a deoxyribofuranose sugar of any of A, G, C, T, or U were present in place of the abasic residue. The abasic residue may incorporate a ribofuranose or deoxyribofuranose ring as in native A, G, C, T, or U. However, the abasic residue does not contain a base or other molecule that can interact with the base on the opposite strand of a duplex which is formed with the abasic residue-containing oligonucleotide. Tetrahydrofuran-type abasic sites and their use as abasic residues are known in the art, as well as their synthesis.

The term "pre-determined nucleic acid sequence", as used herein, refers to a nucleic acid sequence, preferably an RNA or DNA sequence. In particular, the pre-determined nucleic acid sequence, within the present invention, is a sequence that is detectable using the method of the present invention. That is, a nucleic acid sequence available to the skilled person is pre-determined if the skilled person can determine whether the sequence will likely be detectable in a sample obtained from a subject using the methods as provided herein. Within the present invention, the pre-determined nucleic acid sequence comprises at least one probe binding site for the probe that is used in the reagent kit of the invention and at least one primer binding site, preferably (a) binding site(s) for the primer(s) described herein.

The term "hybridisable" or "hybridisation", as used herein, refers to the annealing of complementary nucleic acid molecules. When two nucleic acids "hybridise to" each other, or when one nucleic acid "hybridises to" another, the two nucleic acid molecules exhibit a sufficient number of complementary nucleobases that the two nucleic acid molecules can anneal to each other under the particular conditions (e.g. temperature, salt and other buffer conditions) being utilized for a particular reaction. The most common mechanism of hybridisation involves hydrogen bonding (e.g. Watson-Crick, Hoogsteen or reversed Hoogsteen hydrogen bonding) between complementary nucleobases of the nucleic acid molecules. Hybridisation can occur under varying conditions. Stringent conditions are sequence-dependent and are determined by the nature and composition of the nucleic acid molecules to be hybridised. Nucleic acid hybridisation techniques and conditions are known to the skilled artisan and have been described extensively. See, e.g., Sambrook et al, Molecular Cloning: A Laboratory Manual 2nd ed.. Cold Spring Harbor Press, 1989; Ausubel et al, 1987, Current Protocols in Molecular Biology; Greene Publishing and Wiley-Interscience, New York; Tijessen, 1993, Hybridization with Nucleic Acid Probes, Elsevier Science Publishers, B.V.; and Kricka, 1992, Non-Isotopic DNA Probe Techniques, Academic Press, San Diego, California.

The inventors surprisingly found that, when using a combination of a probe and decoy-oligonucleotide, that have a similar but non-identical binding site, the decoy-oligonucleotide enables an amplification reaction which produces an amplicon that is not hybridisable with the probe, and thus not detected due to the absence of the probe. Therefore, the decoy-oligonucleotide hinders the probe from binding small amounts of the pre-determined nucleic acid sequence. The overall effect of this decoy amplification reaction is the suppression of the detection of carryover contaminants. This mechanism can be exploited in various kinds of nucleotide amplification methods known in the art. In those contexts, the amplification reaction which occurs upon binding of the decoy oligonucleotide produces amplicons which are typically shorter in length relative to the amplification products obtained from the amplification of the pre-determined nucleotide with the primer(s).

The use of this reagent kit therefore ensures the consumption of trace amounts of carryover sequences and reduces the likelihood of false-positive outcomes. This enables a more robust amplification and detection and can reduce the need for laboratory equipment, purification steps or the requirement for a clean working environment.

Accordingly, the invention is at least in part based on the finding that the reagent kit of the invention enables an efficient way of reducing carryover contamination in the amplification of a pre-determined nucleotide sequence, thereby increasing specificity and reducing false-positive outcomes.

The term "carryover" or "carryover contamination", as used herein, refers to an amplification product from a prior assay which contaminates subsequent assays. The contaminant may be transmitted by a technician, an instrument or even via aerosol. In a "negative" sample, where the target nucleic acid is absent, the contaminant creates a false positive result. In a "positive" sample, where the target nucleic acid is present, the contaminant is co-amplified with the true target. Such co-amplification may distort the result of a quantitative assay, where the exact amount of the true target must be determined.

In certain embodiments, the invention relates to the reagent kit of the invention for use in a nucleotide amplification method. In certain embodiments, the invention relates to the use of the reagent kit in a nucleotide amplification method.

In certain embodiments, the invention relates to the reagent kit of the invention, wherein the probe further comprises a label.

The term "label" or grammatical variations thereof, as used herein, refers to any detectable or signal-generating molecule or reporter molecule. These chemical moieties are attached to a nucleotide, nucleotide polymer, or nucleic acid binding factor, wherein the attachment may be covalent or non-covalent. Preferably, the label is detectable and renders the nucleotide or nucleotide polymer detectable to the practitioner of the invention. Detectable labels that may be used in combination with the methods disclosed herein include, for example, a fluorescent label, a chemiluminescent label, fluorescent quenching agents, a radioactive label, coloured molecules or dyes, radioisotopes or scintillants or combinations thereof. Detectable labels also include any useful linker molecule, such as biotin, avidin, streptavidin, HRP, protein A, protein G, antibodies or fragments thereof, Grb2, polyhistidine, Ni2+, FLAG tags, myc tags, heavy metals, enzymes (such as alkaline phosphatase, peroxidase and luciferase), electron donors/acceptors, acridinium esters, dyes and calorimetric substrates. It is also envisioned that a change in mass may be considered a detectable label, as is the case of surface plasmon resonance detection. The person skilled in the art would readily recognize useful detectable labels that are not mentioned above, which may be employed in the operation of the present invention.

The inventors found that the decoy-oligonucleotide is particularly useful, if the probe comprises a label. As such, only amplicons which are hybridisable with the probe are detected due to the presence of the label.

Accordingly, the invention is at least in part based on the finding that a label enables efficient detection of probe binding that can be distinguished from decoy-related binding products.

In certain embodiments, the invention relates to a reagent kit of the invention comprising a larger number of decoy-oligonucleotides than probes.

Within the present invention, it was found that the use of stoichiometric excesses of the decoy-oligonucleotide relative to the probe results in the increased binding of the decoy-oligonucleotide to carryover amplicons, thereby preventing the probe from binding more efficiently and resulting in less false positives. The non-detectable carryover amplicons are formed when the decoy is combined with its opposite primer on the template, and subsequently elongated and amplified to give short amplicons containing mismatch bases to the template, that are not fully hybridisable with the probe. Due to the presence of the mismatch base(s) and limited hybridizability with the probe, the decoy-initiated amplicons produced by this amplification reaction are not bound by the probe and thus are not detected. The detection of the target sequence in the presence of carryover amplicons is ensured as it is relatively abundant in the sample, allowing for probe binding post-amplification of the pre-determined nucleic acid sequence by the added primer(s).

Accordingly, the invention is at least in part based on the finding that a larger decoy oligonucleotide to probe ratio results in more efficient suppression of false positive results.

In certain embodiments, the invention relates to the reagent kit of the invention, wherein the reagent kit comprises one or more DNA primer(s).

The terms "DNA primer" or "primer", as used herein, refer to a nucleic acid molecule comprising a 3'-OH group that, upon hybridisation to a complementary nucleic acid sequence, can be elongated, e.g., via an enzymatic nucleic acid replication reaction. Typically, both the upper and lower limits of the length of the primer are empirically determined.

The primer, probe and decoy oligonucleotide can be matched and fine-tuned to each other, such that the resulting amplicons are optimized, e.g., based on length and binding location. In some embodiments, the primer is part of a single primer nucleic acid amplification method such as a single primer PCR method.

Accordingly, the invention is at least in part based on the finding that an optimized primer location results in higher sensitivity and/or specificity.

In some embodiments, the primer is part of a multiple primer nucleic acid amplification method such as a dual primer nucleic acid amplification method. For example, the primer described herein can be a forward primer or a backward primer. The term "backward primer", as used herein, refers to a primer priming the antisense strand of a DNA sequence to allow the polymerase to extend in one direction along the complementary strand of a DNA sequence. The term "forward primer", as used herein, refers to a primer priming the sense strand of a DNA sequence to allow a polymerase to extend in one direction along one strand of a DNA sequence.

In certain embodiments, the invention relates to the reagent kit of the invention, wherein the reagent kit comprises at least two DNA primers and wherein the binding site of the probe is between the binding site of two DNA primers on the pre-determined nucleotide sequence.

The skilled person is aware that "between the binding site of at least two primers" refers to the binding site that corresponds to a sequence on the pre-determined nucleotide sequence depends on the type of the pre-determined nucleotide sequence. The corresponding sequence may for example be a sequence on the opposite strand (e.g. for double stranded sequences), a complementary sequence (e.g. for RNA/cDNA), or the amplicon of the two primers.

This binding between the binding site of two DNA primers results in shorter amplicons initiated by the decoy oligonucleotide that are more efficiently amplified as they are more likely to be denatured, and hence the competitive amplification of the decoy further results in the faster consumption of amplification reagents. The longer amplicons produced from the amplification of the pre-determined nucleotide sequence with the two primers comprise a sequence that is hybridisable with the probe, and thus can be detected. It was surprisingly found by the inventors that the use of a probe and decoy-oligonucleotide sequence which binds in a region of the amplification product between the two DNA primers has no substantial negative impact on the amplification of the pre-determined nucleotide sequence.

Accordingly, the invention is at least in part based on the finding, that the pre-determined nucleotide sequence can be reliably detected while false positives are efficiently supressed using a decoy-oligonucleotide that binds on a pre-determined nucleotide sequence between two primers.

In certain embodiments the invention relates to a method for detecting the presence of a pre-determined nucleotide sequence in a sample, the method comprising the steps of: (a) adding the reagent kit of the invention; wherein the reagent kit comprises at least one DNA primer; (b) adding one or more enzyme(s) providing activities of DNA polymerase activity and strand-displacement activity to the sample to be analysed for the presence of the pre-determined nucleotide sequence; (c) amplifying the pre-determined nucleotide sequence; and (d) detecting whether an amplification product is present in the sample, wherein presence of the amplification product in the sample is indicative of the presence of the pre-determined nucleotide sequence in the sample.

As used herein, "adding" refers to bringing items in contact. The order of the steps described herein are not indicative of the sequence of addition, but all reagents can be added simultaneously or sequentially. The detection step should be after or during the amplification step.

An enzyme providing activities of RNA- and/or DNA-dependent DNA polymerase activity can synthesize DNA in the 5' to 3' direction based on a template composed of a DNA or RNA strand. As the skilled person is aware, such an enzyme will be successively adding nucleotides to the free 3'-OH of the template. In this regard, the template strand determines the sequence of the added nucleotides based on Watson-Crick base pairing. The activity of the DNA polymerase may be RNA- and/or DNA-dependent. Exemplary polymerases include, but are not limited to Bst DNA polymerase, Vent DNA polymerase, Vent (exo-) DNA polymerase, Deep Vent DNA polymerase, Deep Vent (exo-) DNA polymerase, Bca (exo-) DNA polymerase, DNA polymerase I Klenow fragment, Φ29 phage DNA polymerase, Z-Taq^{™} DNA polymerase, ThermoPhi polymerase, 9°Nm DNA polymerase, and KOD DNA polymerase. See, e.g., U.S. Pat. Nos. 5,814,506; 5,210,036; 5,500,363; 5,352,778; and 5,834,285; Nishioka, M., et al. (2001) J. Biotechnol. 88, 141; Takagi, M., et al. (1997) Appl. Environ. Microbiol. 63, 4504.

The term "strand displacement", as used herein, refers to the ability of an enzyme to separate the DNA and/or RNA strands in a double-stranded DNA molecule and/or in a double-stranded RNA molecule during primer-initiated synthesis. A polymerase with strand-displacing character is specifically required as many substrates are still partially duplex in character.

Detection of the probe-bound amplification product of the pre-determined nucleotide sequence may be performed using any method, including end-point (post-amplification) and real-time (during amplification) methods. One example of end-point detection that is well described in the art is the use of lateral flow assays. Lateral flow devices are particularly advantageous for point-of-care use due to the simplicity of the device, which provides a sensitive and rapid means for the detection of analytes. In general, lateral flow immunoassays are simple one- or two-step assays for the qualitative determination of analytes directly in patient samples. A rapid lateral flow test consists of a system of overlapping porous materials containing the dried components needed to perform the test. These membranes are assembled in small strips, which may be placed into a plastic housing for ease in handling. As is well understood in the art, the combination of an appropriate amplification method and lateral flow analysis permits analyte detection usually in less than 1 hour, achieving limits of detection as low as 1-10 DNA copies. Further end-point detection techniques, such as agarose gel electrophoresis and bridge flocculation assays are suitable and well described in the art, the skilled artisan can easily identify suitable methods. Real-time detection methods such as the use of fluorescent probes and a fluorimeter may also be employed in resource limited settings. Further examples of real-time detection methods are well described in the art.

The inventors found that with the means and methods described herein they achieve better specificity in the amplification of nucleic acids by reducing carryover contaminants. Accordingly, the invention is at least in part based on the improved specificity obtained using the method as described herein. In particular, the means and methods disclosed in the present invention are particularly suitable for use in diagnostic methods utilized in low-equipment environments and point-of-care analysis.

In certain embodiments the invention relates to the method of the invention, wherein amplifying the pre-determined nucleotide sequence comprises incubating the sample resulting from steps (a) and (b) at a fixed temperature.

The term "fixed temperature", as used herein, refers to keeping the temperature condition constant or almost constant so that enzymes and primers can substantially function. The almost constant temperature condition means that not only the set temperature is accurately maintained but also a slight change in the temperature is acceptable within such a degree that it does not spoil substantial functions of the enzymes and primers. For example, a change in temperature of approximately from 0 to 10°C is acceptable.

The amplification of the pre-determined nucleotide sequence under a fixed temperature can be carried out by keeping the temperature at such a level that the activity of the enzyme(s) to be used can be maintained. In addition, in order to effect annealing of the primer(s), probe and decoy-oligonucleotide with the pre-determined nucleotide sequence, the temperature may, for example, be set to the temperature of around the melting temperature (T_{M}) value of the primer(s), probe and decoy-oligonucleotide or lower than that.

Within the present invention, the one or more enzyme(s), DNA primers and the sample to be analysed are incubated in the same tube at a constant temperature. In some embodiments, the temperature is between about 20 and about 50°C. In some embodiments the temperature is more specifically between about 35 and about 45°C. In a further preferred embodiment, the temperature is about 37°C.

This/these temperature (ranges) allow(s) the reaction to take place outside of laboratory settings with low equipment. For example, the reaction can therefore be accelerated by warming the mixture by placing it between hands.

Accordingly, the invention is at least in part based on the finding that the increased reliability provided by the decoy-oligonucleotide is particularly useful in fixed temperature methods, in particular the temperature ranges described herein.

In a further embodiment the invention relates to the method of the invention, wherein at least one enzyme providing activities of single-strand DNA-binding is added.

The term "single-strand DNA-binding enzyme" refers to a protein that binds to a single strand nucleic acid sequence, e.g., an unwound double-stranded nucleic acid sequence. Its main function is to stabilize the single strand nucleic acid sequence during the various exchange transactions that are ongoing in a reaction. Illustrative examples of ssDNA binding proteins are T4 gene 32 protein, E. coli SSB, T7 gp2.5 SSB, and phage phi29 SSB, and ET SSB.

Single-strand DNA binding enzymes are typically used in RPA, therefore this method is particularly applicable to RPA methods. RPA is especially preferred due to its reliability and ease of practice with low equipment and at point-of-care facilities.

Accordingly, the means and methods described herein thus are particularly useful for assays conducted in low equipment environments, where carryover contamination is more likely.

In certain embodiments, the invention relates to the method of the invention, wherein at least two DNA primers are added.

The addition of at least two primer sequences to the reaction mixture facilitates rapid amplification, as each primer sequence matches a sequence on one of the two complementary strands of the pre-determined nucleic acid sequence. Typically, the two primer sequences contain sequences that bind to opposite strands of the template, in this way there is typically a forward primer and reverse primer. Two complementary single strands of DNA are released during denaturation. The forward primer binds to the template DNA, while the reverse primer binds to the other complementary strand, both of which are amplified.

In certain embodiments, the invention relates to the method of the invention or the reagent kit of the invention, wherein at least one DNA primer comprises a label.

The inventors found, that labelling (a) primer(s) can be used, for example, to detect whether a contaminant is present and/or to reduce false positives.

In certain embodiments, the invention relates to a method of the invention, wherein the DNA primer comprises a label which binds to the opposite strand than the probe.

Detection of the desired amplicon produced from the amplification of the pre-determined nucleic acid sequence is facilitated by the addition of a label to at least one of the primers. Where both the probe and its opposite primer sequence contain a label, a dually labelled amplicon is produced. This embodiment confers a further advantage of providing a method amenable to lateral-flow strip detection methods, wherein one label may be used to spatially immobilise the nucleic acid species on a lateral flow membrane via interaction with a moiety immobilised on the membrane, for example via antigen-antibody binding.

Accordingly, the primer label as described herein enables detection and/or increases specificity.

In a further embodiment, the invention relates to the method of the invention, wherein the pre-determined nucleotide sequence comprises a pre-determined DNA sequence.

In a further embodiment, the invention relates to the method of the invention, wherein the pre-determined DNA sequence comprises a sequence comprised in an extrachromosomal DNA sequence, preferably an extrachromosomal DNA sequence encoding a drug susceptibility-related property.

The term "extrachromosomal DNA" as used herein, refers to a deoxyribonucleotide polymer having a chromosomal composition (including histone proteins and/or histone-like proteins) that does not form part of a cellular chromosome. Extrachromosomal DNA molecules have a circular structure and are not linear, as compared to cellular chromosomes.

Extrachromosomal DNA can be isolated by comparably simple setups. These simple setups are typically prone to cross-contamination. The invention provides means and methods provide an effective way to improve that are particularly useful in this context of a simple method setup.

In some embodiments, the drug susceptibility-related property is antibiotic resistance. In certain embodiments, the invention relates to the method of the invention, wherein at least one of the added enzymes provides reverse transcriptase activities.

As used herein, "reverse transcriptase activity" and "reverse transcription" refer to the ability of an enzyme to synthesize a DNA strand (i.e. complementary DNA or cDNA) utilizing an RNA strand as a template. Reverse transcriptase activity may be measured by incubating an enzyme in the presence of an RNA template and deoxynucleotides, in the presence of an appropriate buffer, under appropriate conditions.

As used herein, the term "reverse transcriptase" is used in its broadest sense to refer to any enzyme that exhibits reverse transcription activity as measured by methods known in the art. A "reverse transcriptase" of the present invention, therefore, includes reverse transcriptases from retroviruses, other viruses, as well as a DNA polymerase exhibiting reverse transcriptase activity, such as Tth DNA polymerase, Taq DNA polymerase, Tne DNA polymerase, Tma DNA polymerase, etc. Reverse transcriptase has been used primarily to transcribe RNA into cDNA, which can be used in various amplification methods or further manipulated by methods known in the art.

As used herein, the terms "reverse transcription activity" and "reverse transcriptase activity" are used interchangeably to refer to the ability of an enzyme (e.g., a reverse transcriptase or a DNA polymerase) to synthesize a DNA strand (i.e., cDNA) utilizing an RNA strand as a template

In certain embodiments the invention relates to the method of the invention, wherein the pre-determined nucleotide sequence is comprised in a coronavirus.

The term "coronavirus" refers to a virus of the genus *Coronaviridae. Coronaviridae* is a family of enveloped viruses with a positive-sense single-stranded RNA genome and a helical symmetry. These viruses typically cause respiratory and enteric diseases in humans and/or domestic animals. In addition to causing respiratory and enteric disease, coronaviruses have been associated with pneumonia, exacerbation of asthma, neurological symptoms and myocarditis. Coronaviruses currently known to infect humans are from the alphacoronavirus and betacoronavirus genera. A novel beta-coronavirus, severe acute respiratory syndrome coronavirus-2 (SARS-CoV-2) has caused the coronavirus disease pandemic of 2019 (COVID-19).

Accordingly, the methods of the present invention may be utilised in the diagnosis of infection by a coronavirus, in particular SARS-CoV-2. Rapid diagnosis of infections is highly important in managing symptoms and outbreaks of disease. Furthermore, the use of inexpensive reagents and low-equipment methods, such as the methods disclosed in the present invention, allow for the widespread use of this technology in low-equipment settings.

"a," "an," and "the" are used herein to refer to one or to more than one (i.e., to at least one, or to one or more) of the grammatical object of the article.

"or" should be understood to mean either one, both, or any combination thereof of the alternatives.

"and/or" should be understood to mean either one, or both of the alternatives.

Throughout this specification, unless the context requires otherwise, the words "comprise", "comprises" and "comprising" will be understood to imply the inclusion of a stated step or element or group of steps or elements but not the exclusion of any other step or element or group of steps or elements.

The terms "about" or "approximately", as used herein, refer to "within 20%", more preferably "within 10%", and even more preferably "within 5%", of a given value or range.

The terms "include" and "comprise" are used synonymously. "preferably" means one option out of a series of options not excluding other options. "e.g." means one example without restriction to the mentioned example. By "consisting of' is meant including, and limited to, whatever follows the phrase "consisting of".

Reference throughout this specification to "one embodiment", "an embodiment", "a particular embodiment", "a related embodiment", "a certain embodiment", "an additional embodiment", "some embodiments", "a specific embodiment" or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments. It is also understood that the positive recitation of a feature in one embodiment, serves as a basis for excluding the feature in a particular embodiment.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The general methods and techniques described herein may be performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990).

While embodiments of the invention are illustrated and described in detail in the figures and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

### Brief description of figures

- **Figure 1**: Initial dipstick assays detect unspecific signals in the absence of a decoy-oligonucleotide.
- **Figure 2**: Lowering the concentration of the nfo probe used does not avoid unspecific signals observed on lateral flow dipsticks.
- **Figure 3**: Addition of decoy-oligonucleotide reduces background noise.
- **Figure 4**: Use of two different decoy-oligonucleotides.
- **Figure 5**: The use of decoy primer 2 rejects noise but still allows discrimination of positive samples.
- **Figure 6**: One base mismatch 3' of the probe cleavage site results in carryover suppression.

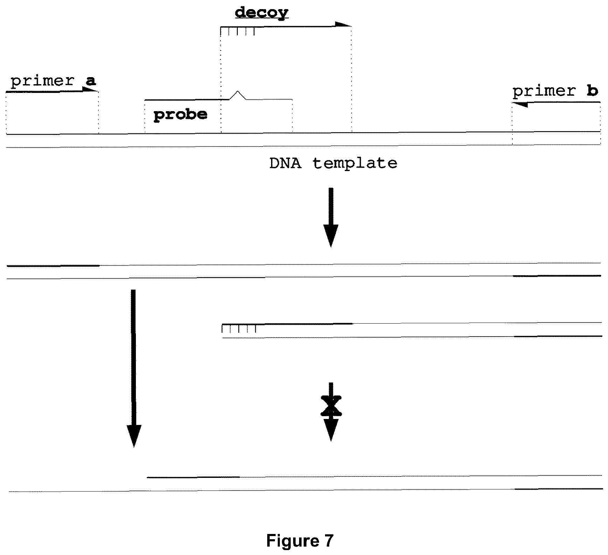

- **Figure 7**: Schematic representation of an embodiment of the invention. Primers a and b are necessary for amplification of target DNA. The probe is necessary for specific detection of an amplicon. The probe has an abasic site for cleavage by an endonuclease, and the probe has no 3' hydroxyl group. A functional decoy-oligonucleotide (1) binds to the same template strand as the probe, (2) has at least one mismatch base adjacent to the abasic site of the probe--marked by vertical lines, (3) has a stretch of bases at its 3' end without mismatches to the template, and (4) has a free 3' hydroxyl group. During exponential amplification, e.g. by RPA, a long amplicon and a competing short amplicon are formed. The probe is only cleaved after incorporation into the long amplicon.

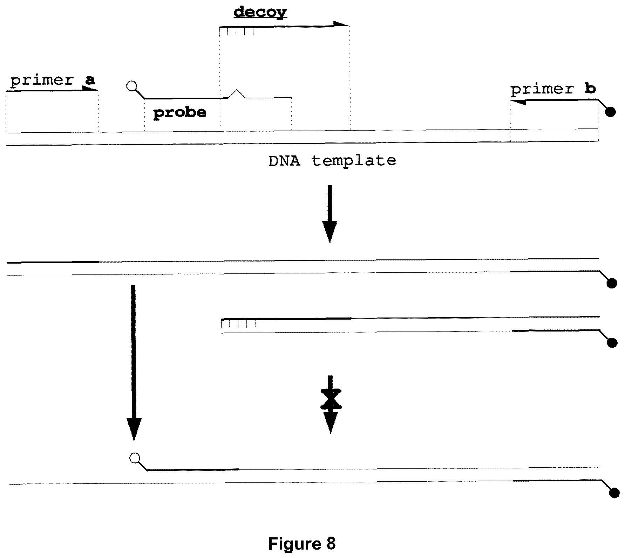

- **Figure 8**: Schematic representation of an embodiment of the invention comprising a dual-labelled system. Here, different labels are introduced via the 5' ends of primer b and of the probe. Only the long amplicon can incorporate the probe and carries both labels.

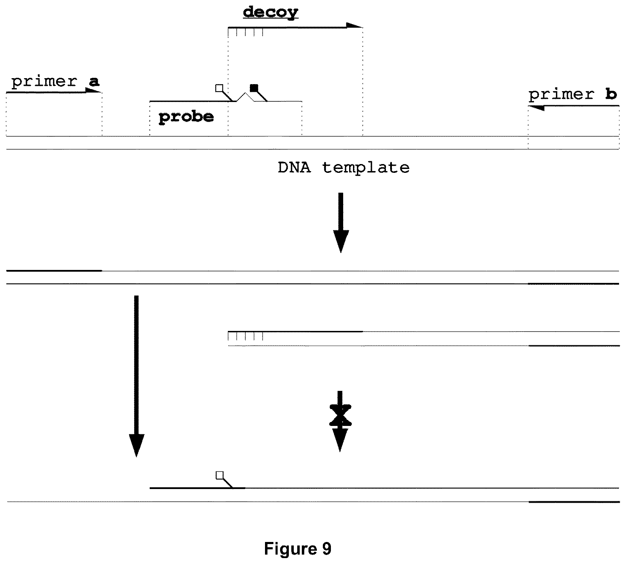

- **Figure 9**: Schematic representation of an embodiment of the invention comprising a FRET based detection system. Here, a fluorescent dye is introduced 5' of the abasic site of the probe, and a quencher is 3' of the abasic site. Quencher and dye are within FRET distance. Only the long amplicon incorporates the probe to relieve quenching.

### Examples

Aspects of the present invention are additionally described by way of the following illustrative non-limiting examples that provide a better understanding of embodiments of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice. However, those of skill in the art should appreciate, in light of the present disclosure that many changes can be made in the specific embodiments which are disclosed and still obtain a like or similar result without departing from the spirit and scope of the invention.

### Materials and Methods

All oligonucleotides were ordered from Integrated DNA Technologies (San Diego, USA) in lyophilised form. Molecular biology grade water (no. W4502 from Sigma, Steinheim, Germany) was used. RPA was carried out with the TwistAmp nfo kit (no. TANF002KIT from TwistDx, Maidenhead, UK). Usually, a reaction volume of 10 µl was preferred. Reactions were prepared as followed in Eppendorf 1.5 ml SafeLock tubes (no. 0030120086, Eppendorf, Hamburg, Germany):
1. A single freeze-dried pellet from the TwistAmp nfo kit was dissolved in 29.5 |jl of rehydration buffer to generate a DNA-free reagent mix.
2. Primers, probe, decoy oligonucleotide, and DNA-containing template solution were mixed in a total volume of 3 |jl per reaction. The final concentrations were 420 nM each for the primers, 120 nM for the probe, and 0-1 |jM for the decoy-oligonucleotide. The template was provided in a volume of 0.5-1 µl.
3. For each reaction, 7 |jl of reagent mix was added to the solution generated in step 2 and mixed thoroughly by pipetting.
4. Immediately thereafter, the tube was closed and incubated at approximately 37°C for an appropriate time (e.g. 9 min).
5. After the defined reaction time, 90 |jl of assay buffer from the HybriDetect 2T kit (no. MGHD2, Milenia Biotec, Giessen, Germany) were directly added to stop the RPA reaction.
6. For lateral flow analysis, a dipstick from the Hybridetect 2T kit was immersed into the solution for each reaction.
7. The readout was taken as a photograph at least one minute after the solvent front had crossed the detection zones of all dipsticks.

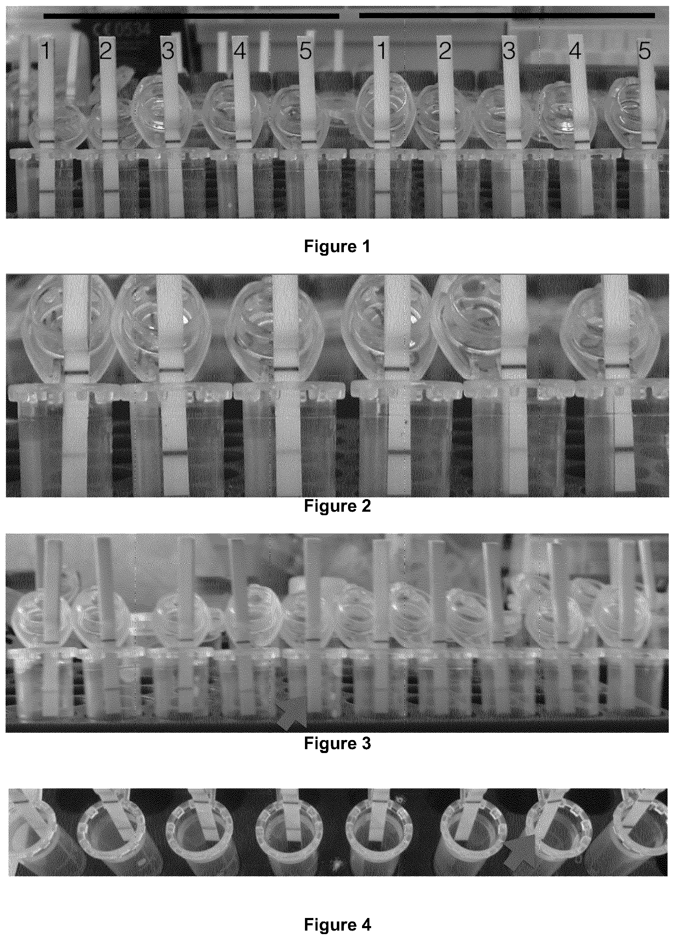

### Example 1: Initial lateral flow dipstick assays detect unspecific signals in the absence of a decoy-oligonucleotide (Figure 1).

Two independent experiments demarked by horizontal bars are shown in Figure 1. RPA was conducted with primers (SEQ ID NO: 2 and SEQ ID NO: 3 with a biotinylation at 5') and probe (SEQ ID NO: 5) specific for SARS-CoV2 S (spike) cDNA (SEQ ID NO: 4) at 37 °C for 10 min. From left to right on Figure 1: Sample 1: Synthetic spike DNA template, 2.5 fg. Sample 2: Spike template plus cDNA from Vero E6 cells (which do not express S mRNA), gene-specific S reverse transcription primer. Sample 3: Water (no template, negative control). Sample 4: Water plus cDNA from Vero E6 cells (gene-specific primer). Sample 5: Water plus cDNA from Vero E6 cells (oligo(dT) and random priming only).

### Example 2: Lowering the concentration of the nfo probe used does not avoid unspecific signals observed on lateral flow dipsticks (Figure 2).

Two cDNA preparations were used (from Vero E6 non-infected and SARS-CoV2-infected cells). A primer complementary to the S mRNA was added before reverse transcription. RPA was conducted with primers (SEQ ID NO: 2 and SEQ ID NO: 3 with a biotinylation at 5') and probe (SEQ ID NO: 5) specific for SARS-CoV2 S cDNA (SEQ ID NO: 4) at 37 °C for 9 min. From left to right on Figure 2: Sample 1: Non-infected, probe concentration 120 nM. Sample 2: Infected, probe concentration 120 nM. Sample 3: Non-infected, probe concentration 60 nM. Sample 4: Infected, probe concentration 60 nM. Sample 5: Non-infected, probe concentration 30 nM. Sample 6: Infected, probe concentration 30 nM.

### Example 3: Addition of decoy-oligonucleotide reduces background noise (Figure 3).

RPA was conducted with primers (SEQ ID NO: 2 and SEQ ID NO: 3 with a biotinylation at 5') and probe (SEQ ID NO: 5) specific for SARS-CoV2 S cDNA (SEQ ID NO: 4) at 37 °C for 9 min. From left to right on Figure 3: Sample 1: No decoy. Sample 2: decoy1 (SEQ ID NO: 11), 1 µM. Sample 3: decoy1 (SEQ ID NO: 11), 0.36 µM. Sample 4: decoy1 (SEQ ID NO:11), 0.1 µM. Sample 5: decoy2 (SEQ ID NO: 6), 1 µM. Sample 6: decoy2 (SEQ ID NO: 6), 0.36 µM. Sample 7: decoy2 (SEQ ID NO: 6), 0.1 µM. Sample 7: decoy3 (SEQ ID NO: 12), 1 µM. Sample 8: decoy3 (SEQ ID NO: 12), 0.36 µM. Sample 9: decoy3 (SEQ ID NO: 12), 0.1 µM.

### Example 4: Use of two different decoy-oligonucleotides (Figure 4).

RPA was conducted with primers (SEQ ID NO: 2 and SEQ ID NO: 3 with a biotinylation at 5') and probe (SEQ ID NO: 5) specific for SARS-CoV2 S cDNA (SEQ ID NO: 4) at 37 °C for 9 min without template unless otherwise noted. From left to right on Figure 4: Sample 1: Water (no decoy). Sample 2: decoy1 (SEQ ID NO: 11), 0.25 µM. Sample 3: decoy1 (SEQ ID NO: 11), 0.5 µM. Sample 4: decoy1 (SEQ ID NO: 11), 1 µM. Sample 5: decoy2 (SEQ ID NO: 6), 0.25 µM. Sample 6: decoy2 (SEQ ID NO: 6), 0.5 µM. Sample 7: decoy2 (SEQ ID NO: 6), 1 µM. Sample 8: decoy2 (SEQ ID NO: 6), 1 µM plus 5 pg synthetic spike DNA template (SEQ ID NO: 1).

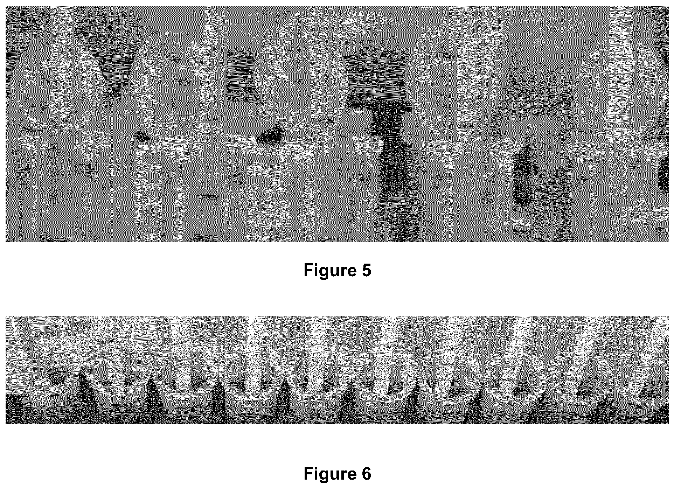

### Example 5: The use of decoy2 rejects noise but still allows discrimination of positive samples (Figure 5).

RPA was conducted with primers(SEQ ID NO: 2 and SEQ ID NO: 3 with a biotinylation at 5') and probe (SEQ ID NO: 5) specific for SARS-CoV2 S cDNA (SEQ ID NO: 4) at 37 °C for 9 min. Decoy2 (SEQ ID NO: 6) was added to all samples at a concentration of 1 µM. From left to right on Figure 5: Sample 1: Water (no template). Sample 2: 5 pg synthetic spike DNA template (SEQ ID NO: 1). Sample 3: 5 fg synthetic spike DNA template (SEQ ID NO: 1). Sample 4: cDNA from non-infected Vero E6 cells. Sample 5: cDNA from SARS-CoV2-infected Vero E6 cells.

### Example 6: One base mismatch 3' of the probe cleavage site can result in full carryover suppression (Figure 6).

RPA was conducted with primers (SEQ ID NO: 2 and SEQ ID NO: 3 with a biotinylation at 5') and probe (SEQ ID NO: 5) specific for SARS-CoV2 S cDNA (SEQ ID NO: 4) at 37 °C for 9 min. There was either no template (samples 1-5) or cDNA synthesised from an RNA sample of a SARS-CoV2-infected patient (samples 6-10. Decoy oligonucleotide concentrations were 1 µM. From left to right on Figure 6: Sample 1: No template, no decoy. Sample 2: decoy2c (SEQ ID NO: 7), one mismatch 3' of the cleavage site, 5' not covered. Sample 3: decoy2d (SEQ ID NO: 8), no mismatch 3' of the cleavage site, 5' not covered. Sample 4: decoy2e (SEQ ID NO: 9), binds one base 3' of the cleavage site, 5' not covered. Sample 5: decoy2f (SEQ ID NO: 10), binds two bases 3' of the cleavage site. Sample 6: no decoy. Sample 7: decoy2c (SEQ ID NO: 7). Sample 8: decoy2d (SEQ ID NO: 8). Sample 9: decoy2e (SEQ ID NO: 9). Sample 10: decoy2f (SEQ ID NO: 10).

## Claims

1. A reagent kit comprising a decoy-oligonucleotide and a probe, wherein
a) the probe comprises a cleavage site and a binding site hybridisable to a pre-determined nucleotide sequence; and
b) the decoy-oligonucleotide comprises a 3'-OH group, at least one mismatch base compared to the binding site of the probe.

2. The reagent kit of claim 1, wherein the probe further comprises a label.

3. The reagent kit of claim 1 or 2, wherein the reagent kit comprises a larger number of decoy-oligonucleotides than probes.

4. The reagent kit of any one of claims 1 to 3, wherein the reagent kit comprises one or more DNA primer(s).

5. The reagent kit of claim 4, wherein the reagent kit comprises two or more DNA primers and wherein the binding site of the probe is between the binding site of two DNA primers on the pre-determined nucleotide sequence.

6. A method for detecting the presence of a pre-determined nucleotide sequence in a sample, the method comprising the steps of:
(a) adding the reagent kit of any one of claims 1 to 5; wherein the reagent kit comprises at least one DNA primer;
(b) adding one or more enzyme(s) providing activities of DNA polymerase activity and strand-displacement activity to the sample to be analysed for the presence of the pre-determined nucleotide sequence;
(c) amplifying the pre-determined nucleotide sequence; and
(d) detecting whether an amplification product is present in the sample,
wherein the presence of the amplification product in the sample is indicative of the presence of the pre-determined nucleotide sequence in the sample.

7. The method of claim 6, wherein amplifying the pre-determined nucleotide sequence comprises incubating the sample resulting from steps (a) and (b) at a fixed temperature.

8. The method of claim 7, wherein the fixed temperature is between 20 and 50°C, preferably between 35 and 45°C.

9. The method of any one of claims 6 to 8, wherein at least one enzyme providing activities of single-strand DNA-binding is added.

10. The method of any one of claims 6 to 9, wherein at least two DNA primers are added.

11. The method of any one of claims 6 to 11, wherein at least one of the DNA primers comprises a label.

12. The method of claim 11, wherein the DNA primer comprising a label which binds to the opposite strand than the probe.

13. The method of any one of claims 6 to 12, wherein the pre-determined nucleotide sequence comprises a pre-determined DNA sequence.

14. The method of claim 13, wherein the pre-determined DNA sequence comprises a sequence comprised in an extrachromosomal DNA sequence, preferably an extrachromosomal DNA sequence encoding a drug susceptibility-related property.

15. The method of any one of claims 6 to 12, wherein at least one of the added enzymes provides reverse transcriptase activities, preferably wherein the pre-determined nucleotide sequence is comprised in a coronavirus.
